# EUROPEAN PATENT APPLICATION

(11) **EP 3 915 585 A1**
(43) Date of publication of application: **01.12.2021**
(21) Application number: 20176521.1
(22) Date of filing: 26.05.2020
(51) Int. Cl.: A61K 45/06, A61K 31/585, A61K 39/395

(54) **THERAPEUTIC COMBINATIONS COMPRISING AGONISTS OF FERROPTOSIS FOR TREATING PROLIFERATIVE DISORDERS**

(71) Applicant: Chemotherapeutisches Forschungsinstitut Georg-Speyer-Haus, 60596 Frankfurt am Main (DE); Johann Wolfgang Goethe-Universität Frankfurt, 60323 Frankfurt am Main (DE)
(72) Inventor: GRETEN, Florian, 61352 Bad Homburg (DE); CONCHE, Claire, 60596 Frankfurt am Main (DE); FINKELMEIER, Fabian, 60318 Frankfurt am Main (DE); NICOLAS, Adele, 65451 Kelsterbach (DE)
(74) Representative: Kuttenkeuler, David

(57) **Abstract**

The invention is based on the finding that ferroptosis mediated cell death results in the local recruitment of immune cells and that a concomitant sensitization of tumour tissue and tumour cells to a cell-mediated immune response results in synergistic therapeutic effects against proliferative diseases. By inducing or supporting ferroptosis, such as inhibition of its key regulators, it could be shown that tumour cells become sensitized to immune checkpoint inhibition, which was even further improved when also myeloid cell recruitment is blocked. Hence, the enhanced recruitment of immune cells via ferroptosis can be harnessed for antitumour response by immune checkpoint inhibition and/or suppression of local recruitment of immune suppressive cell fractions. The invention provides such combinatorial approaches to treat proliferative disorders, preferably solid cancers which are characterized by a resistance to immune checkpoint inhibition, which often is observed in hepatocellular carcinoma. The invention pertains to the medical application of ferroptosis induction using ferroptosis agonists in combination with immune checkpoint inhibition - such as inhibition of the PD-1/PD-L1 axis - in the treatment and prevention of proliferative disorders. The invention provides compounds and compositions for use in the indicated medical approaches.

## Description

### FIELD OF THE INVENTION

The invention is based on the finding that ferroptosis mediated cell death results in the local recruitment of immune cells and that a concomitant sensitization of tumour tissue and tumour cells to a cell-mediated immune response results in synergistic therapeutic effects against proliferative diseases. By inducing or supporting ferroptosis, such as inhibition of its key regulators, it could be shown that tumour cells become sensitized to immune checkpoint inhibition, which was even further improved when also myeloid cell recruitment is blocked. Hence, the enhanced recruitment of immune cells via ferroptosis can be harnessed for anti-tumour response by immune checkpoint inhibition and/or suppression of local recruitment of immune suppressive cell fractions. The invention provides such combinatorial approaches to treat proliferative disorders, preferably solid cancers which are characterized by a resistance to immune checkpoint inhibition, which often is observed in hepatocellular carcinoma. The invention pertains to the medical application of ferroptosis induction using ferroptosis agonists in combination with immune checkpoint inhibition - such as inhibition of the PD-1/PD-L1 axis - in the treatment and prevention of proliferative disorders. The invention provides compounds and compositions for use in the indicated medical approaches.

### DESCRIPTION

Hepatocellular Carcinoma (HCC) is the 6th most common malignancy in the western word while being the 3rd most common cause of cancer related death. HCC is a heterogeneous disease known to be resistant to conventional radio- and chemo-therapies. Immunotherapy had just been FDA-approved for second-line treatment based on clinical trial with a 17% overall response¹. Therefore, HCC highlights the need for finding new and innovative therapeutic approaches for cancer treatment.

Ferroptosis is a programmed necrosis whose execution requires the accumulation of cellular reactive oxygen species (ROS) in an iron-dependent manner. Ferroptosis can be triggered by inhibiting the activity of cystine-glutamate antiporter, leading to the depletion of glutathione (GSH), the major cellular antioxidant whose synthesis requires cysteine. As such, ferroptosis is caused by the loss of cellular redox homeostasis. Further, it appears that lipid ROS/peroxides instead of mitochondrial ROS play more crucial roles in ferroptosis, and inactivation of glutathione peroxidase 4 (GPX4), an enzyme required for the clearance of lipid ROS, can induce ferroptosis even when cellular cysteine and GSH contents are normal. It is further understood that the intracellular metabolic pathway glutaminolysis also plays crucial roles in ferroptosis by promoting cellular ROS generation.

The GPx4 comprises an important constituent of the cellular antioxidant network(1) that prevents lethal lipid peroxidation and ferroptosis, a recently described iron-dependent non-apoptotic form of cell death(2). Ferroptosis has been suggested to be involved in the pathogenesis of neurodegenerative diseases and ischemic injuries while it can also confer tumour suppressive activity(3,4,5). Suppression of system xc- expression by the tumour suppressors p53- and BAP1, or by IFNγ released by CD8+ T cells sensitize tumour cells to ferroptosis(6,7,8).

Recently, immunotherapy targeting immune checkpoint signaling pathways has been shown to be effective in treating cancer. These pathways suppress immune responses and are crucial for maintaining self-tolerance, modulating the duration and amplitude of physiological immune responses in peripheral tissues, and minimizing collateral tissue damage. It is believed that tumour cells can activate the immune checkpoint signaling pathways to decrease the effectiveness of the immune response against tumour tissues. Many of these immune checkpoint signaling pathways are initiated by interactions between checkpoint proteins present on the surface of the cells participating in the immune responses, e.g., T cells, and their ligands, thus they can be readily blocked by agents or modulated by recombinant forms of the checkpoint proteins or ligands or receptors. The agents blocking the immunosuppression pathway induced by checkpoint proteins are commonly referred to as checkpoint inhibitors and a few have been commercialized. Cytotoxic T-lymphocyte-associated antigen 4 (CTLA4) antibodies, blocking the immunosuppression pathway by the checkpoint protein CTLA4, were the first of this class of immunotherapeutics to achieve US Food and Drug Administration (FDA) approval. Clinical findings with blockers of additional immune-checkpoint proteins, such as programmed cell death protein 1 (PD-1) or its ligand PD-L1, indicate broad and diverse opportunities to enhance anti-tumour immunity with the potential to produce durable clinical responses.

WO/2019/113004 discloses methods of treatment by ferroptotic induction, as well as compositions and dosing regimens that are part of such methods, and combination therapies that include multiple administration steps whereby a ferroptosis-inducing agent is administered sometime after hormone therapy in a patient is initiated.

Thus, it is an objection of the invention to provide novel approaches utilizing immunemodulatory treatments such as checkpoint inhibition as an avenue for the treatment of cancer diseases.

### BRIEF DESCRIPTION OF THE INVENTION

Generally, and by way of brief description, the main aspects of the present invention can be described as follows:
In **a first aspect,** the invention pertains to an agonist of ferroptosis for use in the treatment of a proliferative disorder in a subject, wherein the treatment involves agonising ferroptosis by administering the agonist of ferroptosis to the subject, and sensitizing cells involved with the proliferative disorder to a cell mediated immune response, for example by administering an immune checkpoint inhibitor to the subject.
In **a second aspect,** the invention pertains an agonist of ferroptosis for use in the treatment of a proliferative disorder, in a subject, the treatment comprising exposing tissue or cells involved with the proliferative disorder in the subject to: (i) an immune checkpoint inhibitor, and (ii) the agonist of ferroptosis, and, optionally, (iii) a blocker of myeloid cell recruitment.
In **a third aspect,** the invention pertains to an agonist of ferroptosis for use in aiding a treatment with an immune checkpoint inhibitor in a subject, by enhancing susceptibility of cells or tissue involved with a proliferative disorder to the treatment with the immune checkpoint inhibitor.
In **a fourth aspect,** the invention pertains to a method of treating a disease in a subject in need of the treatment, the method comprising the steps of administering to the subject a therapeutically effective amount of an agonist of ferroptosis, wherein the treatment involves agonising ferroptosis in the subject and sensitizing cells involved with the proliferative disorder to a cell mediated immune response.
In **a fifth aspect,** the invention pertains to a method of treating a proliferative disorder in a subject in need of the treatment, the method comprising the concomitant or sequential administration of (i) an agonist or inducer of ferroptosis, (ii) an immune checkpoint inhibitor, and, optionally (iii) a blocker of myeloid cell recruitment.

### DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to an agonist of ferroptosis for use in the treatment of a proliferative disorder in a subject, wherein the treatment involves agonising ferroptosis and sensitizing cells involved with the proliferative disorder to a cell mediated immune response, preferably wherein the cell-mediated immune response is induced by ferroptosis; and wherein the treatment comprising administering to the subject the agonist of ferroptosis.

In an alternative or additional aspect, the invention pertains to methods of treatment of a proliferative disorder, or compounds or compositions for use in such methods of treatment of a proliferative disorder, in a subject in need of the treatment, comprising the concomitant or sequential administration of
- An inducer or agonist of ferroptosis,
- An immune checkpoint inhibitor and/or a blocker of myeloid cell recruitment.

In particular embodiments of the invention the treatment involves agonising ferroptosis and (thereby) inducing a cell-mediated immune response in cells or tissue involved with, or surrounding, the proliferative disorder.

In context of the herein disclosed invention, the term "agonist of ferroptosis", shall be understood to refer to any compound or means that when brought into contact with a cell induces ferroptosis in that cell, and/or a compound when administrated to a subject result in the induction of, or in the increase of, ferroptotic cell death. Many compounds are described to have an activity as inducer or enhancer of ferroptosis, and such compounds, known to the skilled person, shall be encompassed by the present invention. In preferred embodiments, the agonists of ferroptosis are selected from the group consisting of (i) RSL3 and/or other GPX4 inhibitor or antagonist (such as withaferin A(WA)), (ii) erastin (e.g., and/or another compound which inhibits amino acid transporters system xc-), and (iii) buthionine sulfoximine (BSO) which inhibits gamma-glutamylcysteine synthetase and production of glutathione, or (iv) activators of the nuclear factor-like 2 pathway, for example through targeting of Kelch-like ECH-associated protein 1.

Agonists or inducers of ferroptosis which are small molecule inducers are disclosed in WO 2018/118711, which is incorporated herein by reference in its entirety, and in particular the compounds disclosed in paragraphs [112] to [126] of WO 2018/118711.

Agonists or inducers of ferroptosis which are small molecule inducers are disclosed in WO/2019/106434, which is incorporated herein by reference in its entirety, and in particular the compounds disclosed on pages 15 and 16, and in particular such activators which are provided in table 1, according to the shown IC₅₀ cell viability results of table 2 of the document.

In addition, a ferroptosis agonist may be a compound selected from an antibody, or other antigen binding compound, a T-cell receptor, a nucleic acid inhibitor.

Induction of ferroptosis in context of the herein disclosed invention includes the induction of ferroptotic cell death in the cancer cells and/or the surrounding tissue, such as healthy cells or tissue. Hence, unlike the prior art, the present invention in some embodiments seeks no selective cancer ferroptosis induction, but activating or promoting ferroptosis in addition in non-cancerous, but tumour surrounding cells.

Agonists or inducers of ferroptosis are described in, among others, Weiwer et al, 2012, Bioorg Med Chem Lett. 22(4):1822-1826; and Bittker et al.,"Screen for RAS-Selective Lethal Compounds and VDAC Ligands- Probe 1," Probe Reports from the NIH Molecular Libraries Program [Internet], Bethesda (MD), National Center for Biotechnology Information (US); 2010-. 2010 Mar 26 [updated 2011 Feb 10], PMID: 21634081; all publications incorporated herein by reference.

In some embodiments, the agonists or inducers of ferroptosis, such as those described herein, include RSL3, erastin and erastin derivatives (MEII, PE, AE), ML210 (DPI10), ML162 (DPI7), auranofin, sulfasalazine, artesunate, artemisinin, dihydroatemisinin, artemether, and other artemisinin derivatives, sorafenib and sorafenib analogs (SRS13-45 and SRS13-60), buthionine sulfoximine (BSO), altretamine, almitrine, lanperisone, DPI2, DPI12, DPI13, DPI17, DPI18, DPI19,31MEW44, and ML160.

In some embodiments, the agonists or inducers of ferroptosis can be an inhibitory nucleic acid targeting expression of a cellular molecule involved in regulating ferroptosis. Nucleic acids, such as shRNA and anti-sense nucleic acids can target cellular components, such as cysteine-glutamate antiporter (system X- c), glutathione peroxidase 4 (GPX4), NOX, and ALOX15. shRNAs and inhibitory RNAs for such cellular targets are described in patent publications US20150079035, US20150175558, US20100081654, WO2015051149, WO2015084749, WO2013152039 and WO2015109009; all publications incorporated herein by reference. Other cellular targets and corresponding inhibitory nucleic acids are described in Gao et al., 2016, Cell Res.26:1021-1032, incorporated herein by reference.

The agonist of ferroptosis is in preferred embodiments administered in a therapeutically effective amount that is effective to induce or enhance ferroptosis in a cell or tissue involved with the proliferative disorder; preferably by inhibiting GPX4 in the cells involved with, or surrounding cells of, the proliferative disorder.

In some preferred embodiments of the present invention, the agonist of ferroptosis is Withaferin A, which is a plant derived steroidal lactone ((4β,5β,6β,22β)-4,27-Dihydroxy-5,6:22,26-diepoxyergosta-2,24-diene-1,26-dione).

A therapeutic treatment in context of the invention is based on a combinatorial approach of inducing or promoting ferroptosis in the cancer or tumour environment in a subject together with the use of checkpoint inhibition in order to sensitize the tumour cells to the cell mediated immune response initiated by the ferroptotic cell death. The invention therefore provides a treatment comprising inducing or supporting ferroptosis together with a sensitization of cancer cells to a cell-mediated immune response, for example by immune checkpoint inhibition and/or suppression of recruitment of immune suppressive components, wherein the cell-mediated immune response induces killing of cells involved with the proliferative disorder.

The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell, and in particular (such as when an increase in cell-mediated immune response is required, such as to treat a cancer) the T cell can be a cytotoxic T cell (also known as TC, cytotoxic T lymphocyte, CTL, T-killer cell, cytolytic T cell, CD8+ T-cell or killer T cell). A CTL is a T-cell that is involved in the killing of cancer cells, cells that are infected (particularly with viruses), or cells that are damaged in other ways. Other preferred immune cells for such embodiments can include Tumour-Infiltrating Lymphocytes (TILs). TILs are white blood cells that have left the bloodstream and migrated into a tumour. Typically, TILs are a mix of different types of cells (i.e., T cells, B cells, NK cells, macrophages) in variable proportions, T cells being the most abundant cells. TILs can often be found in the stroma and within the tumour itself, and are implicated in killing tumour cells. The presence of lymphocytes in tumours is often associated with better clinical outcomes.

The term "cell-mediated immune response", as used herein, may include, but is not limited to, a response in a host organism involving, utilising, and/or promoting any one or combinations of T cell maturation, proliferation, activation, migration, infiltration and/or differentiation, and/or the activation/modulation/migration/infiltration of a macrophage, a natural killer cell, a T lymphocyte (or T cell), a helper T lymphocyte, a memory T lymphocyte, a suppressor T lymphocyte, a regulator T lymphocyte, and/or a cytotoxic T lymphocyte (CTL), and/or the production, release, and/or effect of one or more cell-secretable or cell-secreted factor such as a cytokine or autocoid (in particular a pro-inflammatory cytokine such as TNF), and/or one or more components of any of such processes (such as a cytokine or autocoid, particular a pro-inflammatory cytokine such as TNF). The term "cell-mediated immune response," as used herein, may include a cellular response involving a genetically engineered, in-vitro cultured, autologous, heterologous, modified, and/or transferred T lymphocyte, or it may include a cell-secretable or cell-secreted factor (such as a cytokine or autocoid) produced by genetic engineering. A cell-mediated immune response is preferably not a humoral immune response, such as an immune response involving the release of antibodies. In certain embodiments, in particular when the proliferative disorder is a cancer or tumour, the cell-mediated immune response is an anti-tumour cell-mediated immune response. For example, one that leads to a reduction in tumour (cell) growth, such as a cytotoxic cell-mediated immune response (such as a cytotoxic T cell) that kills cells of the cancer or tumour.

The agonist of ferroptosis according to the invention is administered to the subject to elicit an immunological environment around cells involved with the proliferative disorder, which can be transferred by concomitant checkpoint inhibition to an immune cell mediated killing, such as by natural killer (NK) cells or T cells. The treatment of the invention is mediated by the induction or promotion of ferroptosis in combination with checkpoint inhibition. In context of the herein disclosed invention cells surrounding a cell or tissue involved with the proliferative disorder are cells of the microenvironment of the diseased tissue, such a tumour microenvironment.

Therefore, the invention is particular useful in the context of the use of checkpoint inhibitor therapy. As such, and as a preferred embodiment of the invention, the subject receives or received a prior therapy with a checkpoint inhibitor. Alternatively, the present invention pertains to a treatment which further comprises the administration of an immune checkpoint inhibitor to the subject, either concomitantly or sequentially, with the agonist of ferroptosis.

The term "checkpoint inhibitor" or "immune checkpoint inhibitor", as used herein, refers to an agent used in cancer immunotherapy. A checkpoint inhibitor blocks an inhibitory immune checkpoint and thus restores immune system function, for example, an inhibitor of the immune checkpoint molecule CTLA-4, such as ipilimumab, or an inhibitor of PD-1, such as nivolumab or pembrolizumab, or an inhibitor of PD-Li, such as atezolizumab, avelumab, and durvalumab. In many of the embodiments, a checkpoint inhibitor relates to an antibody which targets a molecule involved in an immune checkpoint. The immune checkpoint inhibitor is selected from a immunotherapy comprising (administration of) an antibody binding to an immune checkpoint molecule, a non-antibody peptide or a small molecule, targeting and inhibiting CTLA-4, PD-1 or PD-L1.

The agonist of ferroptosis in further preferred embodiments may include the concomitant or sequential use of a suppressor of the recruitment of immune suppressive cell fractions, such as by the use of a blocker of myeloid cell recruitment.

The agent that is intended to reduce the myeloid cell recruitment, preferably myeloid derived suppressor cells (MDSC), can be any agent that can selectively recognize such cells, and directly or indirectly deplete their numbers and/or inhibit their recruitment to the tumour microenvironment, preferably a recruitment induced by ferroptosis. Preferred agents include those that specifically recognize any pan-myeloid marker. As such, the anti-MCA can be a mAb that can specifically recognize myeloid cells, such as by specifically binding to any myeloid cell surface marker. In certain embodiments, the anti-MCA can be used to deplete myeloid cells by specifically binding to them, resulting in their selective removal/destruction via the endogenous activity of the immune system of the individual to whom the anti-MCA is administered. In other embodiments, the anti-MCA can be conjugated to a cytotoxic moiety, such that targeted delivery of the anti-MCA results in death of the cells via at least in part by activity of the cytotoxic agent. In another embodiment, this invention may impede myeloid cell trafficking from the circulation to the tumour microenvironment. In an embodiment, the anti-MCA is a mAb that specifically recognizes either component, or a complex of Mac-1, which is composed of CD11b and CD18.

With respect to compounds and compositions suitable as suppressors of MDSC herein, it should be noted that any reagents and/or formulations that inhibit MDSCs, IL-8-mediated signaling, CXCR1 pathway activity, and/or CXCR2 pathway activity are contemplated, including but not limited to an HMGB1 antibody or the compound SB225002. For example, with respect to contemplated inhibitors of MDSCs, it is preferred that the reagent(s) can preferentially, or even selectively, inhibit granulocytic MDSC. Yet, it is also contemplated that any reagents that can effectively inhibit any other types of MDSCs in the tumour and/or tumour microenvironment can be used. In addition, it should be appreciated that suitable reagent(s) can act in various manners, including inhibiting recruitment of MDSCs to the tumour, inhibiting expansion of MDSC in the tumour, inhibiting differentiation of MDSCs, and/or inhibiting activity (e.g., secreting chemokines, etc.) at the tumour, or even eliminating or reducing the number of MDSCs in the tumour or tumour microenvironment. For example, MDSC inhibitors may reduce or abrogate recruitment of MDSCs to the tumour and/or accumulation of MDSCs in the tumour, which may be achieved by administration of one or more antagonists of one or more colony-stimulating factor 1 receptor (CSF-R), granulocyte colony-stimulating factor (G-CSF), C-C motif chemokine ligand 2 (CCL2), or C-X-C chemokine receptor type 4 (CXCR4). As used herein, antagonist refers any molecule that is capable to directly or indirectly inhibit the activity of target molecule. Thus, antagonist may include small molecule inhibitors, antibodies or fragments thereof that bind to the target molecule, single-chain variable fragment (scFv) molecule binding to the target molecule, or any other suitable binding molecules. For example, the antagonist of CSF-R may include a small molecule inhibitor (e.g., Pexidartinib, etc.) or one or more monoclonal antibodies against CSF-R (e.g., Emactuzumab, AMG820, imc-CS4, MCS110, etc.).

Preferred MDSC antagonists which are CXCR2- and/or CXCR1/2 antagonist in context of the invention are AZ5069, Danirxin (GSK 1325756), Navarixin (MK7123). Additional preferred MDSC antagonists are Liver-X nuclear receptor (LXR) agonist GW3965 (Tavazoie MF et al Cell. 2018 Feb 8; 172(4): 825-840.e18), or RGX-104.

Preferred additional compounds shown to have an antagonistic effect on MDSC function are PERK inhibitors AMG-44, GSK-2606414; tauroursocholic acid (TUCA); Phosphodiesterase-5 inhibitor sildenafil and tadalafil (J Exp Med (2006) 203 (12): 2691-2702); IL-1β antagonist recombinant IL-1RA (Cancer Cell. 2008 Nov 4; 14(5): 408-419); IL6 (Cheng L et al. 2010 PLoS ONE 6(3): 617631); IL-18 antagonist (J Immunol December 1, 2014, 193 (11) 5453-5460;) and the TNF-α antagonist etanercept (Immunity Volume 38, Issue 3, 21 March 2013, Pages 541-554); the macrophage migration inhibitory factor (MIF) antagonist sulforaphane (J Immunol December 15, 2012, 189 (12) 5533-5540); the Ido inhibitor indoximod (Cell Rep. 2015 Oct 13; 13(2): 412-424.); SIRT1, HIF-1α inhibitor (Cellular & Molecular Immunology volume 15, pages618-629(2018) and CancerRes; 74(3); 727-37.2013 AACR.); Nitroaspirin (PNAS March 15, 2005 102 (11) 4185-4190); Triterpenoid (DOI: 10.1158/1078-0432.CCR-09-3272 Published March 2010); a Cox2 inhibitor and PGE2 antagonist, EP2 and EP4 antagonists, celecoxib (Blood (2011) 118 (20): 5498-5505; J Exp Med (2005) 202 (7): 931-939); a MEK inhibitor (Nature Communications volume 11, Article number: 2176 (2020)).

Alternatively, or additionally, expansion of the MDSCs in the tumour may be inhibited by administering gemcitabine, amino bisphosphonates, sunitinib, or celecoxib, and differentiation of MDSCs in the tumour may be inhibited by taxanes, curcumin, or Vitamin D3. In addition, MDSC activity in the tumour may be inhibited by administration of amiloride, CpG, COX2 inhibitors, PDE-5 inhibitors, or PGE2 inhibitors. Further, MDSCs in the tumour or tumour microenvironment can be eliminated or at least reduced by treating the tumour with doxorubicin (or aldoxorubicin for enhanced activity in the acidic tumour microenvironment). As used herein, the term "administering" the formulation refers to both direct and indirect administration of the formulation, wherein direct administration of the formulation is typically performed by a health care professional (e.g., physician, nurse, etc.), and wherein indirect administration includes a step of providing or making available the formulation to the health care professional for direct administration (e.g., via injection, etc.).

In some embodiments, it is contemplated that different types of MDSC inhibitors can be administered together to act on the MDSCs in different manner, preferably at different time points. For example, inhibitors for recruitment/accumulation of MDSCs and inhibitors of MDSC activity can be coupled with different types of carrier (e.g., albumin-linked, encapsulated in a lipid micelle, cell-penetrating peptide-linked, etc.) such that the inhibitors for recruitment and/or accumulation of MDSCs acts on the tumour prior to the inhibitors of MDSC activity by differential access rate to the tumour or different diffusion rate of the reagents. In this scenario, it is contemplated that the recruitment of the MDSCs to the tumour is blocked first and then the activity of pre-existing MDSCs in the tumour is blocked such that the effect of MDSC activity in the tumour can be effectively eradicated. In other embodiments, different types of MDSC inhibitors can be administered by different methods of administration to act on the MDSCs at different time points. For example, the inhibitors for recruitment/accumulation of MDSCs can be injected intratumourally (e.g., especially where the tumour is a solid tumour, and the tumour cell is from the solid tumour) and the inhibitors of MDSC activity can be injected intravenously (or any other systemic injection).

The herein disclosed invention in the various aspects and embodiments suggest a combinatorial treatment regime for cancer therapy. In such aspects and embodiments combination of the following agents is most preferred; Withaferin A+ (as agonist of ferroptosis), an PD1 antagonist, such as preferably an anti-PDi antibody (as immune checkpoint inhibitor), and as an MDSC antagonist any of the following compounds: AZ5069, Danirixin, Navarixin or RGX-104.

The first aspect in particular relates to a treatment that does not necessarily kills or reduces cells involved with proliferative disorders, such as cancer cells, directly, but as a concomitant or preparatory treatment, to enhance or support the actual treatment of the disease. Such treatment may be independent or part of the present invention, and includes immune therapy such as administering a cancer vaccine (e.g., viral vaccine, bacterial vaccine, yeast vaccine, etc.), administering one or more immune-stimulatory molecules (e.g., CD80, CD86, CD30, CD40, CD30L, CD40L, ICOS-L, B7-H3, B7-H4, CD70, OX40L, 4-1BBL, GITR-L, TIM-3, TIM-4, CD48, CD58, TLiA, ICAM-1, and LFA3, etc.), immune stimulatory cytokines (e.g., IL-2, IL-12, IL-15, IL-15 super agonist (ALT803), IL-21, IPS1, and LMP, etc.), and/or checkpoint inhibitors (e.g., antibodies or binding molecules to CTLA-4 (especially for CD8+ cells), PD-1 (especially for CD4+ cells), TIM1 receptor, 2B4, and CD160, etc.). In addition, contemplated immune therapies include any cell-based therapies such as administration of NK cells, genetically engineered NK cells, and especially aNK cells, haNK cells, optionally with bound antibody, or taNK cells, NKT cells, genetically engineered NKT cells, (re-)activated T cells or T cells expressing a chimeric antigen receptor, and/or dendritic cells expressing cancer neoepitopes or cancer specific or associated antigens. In addition, the treatment may comprise least one or more cancer therapies including chemotherapy, a radiation therapy, or immune therapy after preconditioning the tumour or tumour environment with agonists of ferroptosis, optionally together with MSDC inhibitors. The treatment regimen and schedule may vary depending on the type(s) of preconditioning and cancer therapies. For example, a chemotherapy or a radiation therapy may be administered to a patient at least 1 day, 3 days, 5 days, 7 days after completing preconditioning of the tumour. In another example, in some embodiments, a cell-based immune therapy may be administered to a patient at least 1 day, 3 days, 5 days, 7 days after completing preconditioning of the tumour. In still other embodiments, depending on the type of cell-based immune therapy, the cell-based immune therapy may be administered to the patient by completion of preconditioning of the tumour or even during the preconditioning of the tumour (e.g., at least 12 hours, at least 1 day, at least 3 days after beginning of preconditioning, etc.).

The treatment of the invention may further comprise the administration of at least one additional anti-proliferative therapeutic such as a chemotherapeutic agent.

A proliferative disorder in context of the invention in all aspects and embodiments is preferably a cancer or tumour disease. As used herein, "cancer" can include the term "solid tumour." As used herein, the term "solid tumour" refers to those conditions, such as cancer, that form an abnormal tumour mass, such as sarcomas, carcinomas, and lymphomas. Examples of solid tumours include, but are not limited to, hepatocellular carcinoma, colorectal cancer and the like. In certain embodiments, the solid tumour disease can be, for example, an adenocarcinoma, squamous cell carcinoma, large cell carcinoma, and the like.

In certain embodiments, the cancer can be, for example, esophageal cancer, gastroesophageal junction cancer, gastroesophageal adenocarcinoma, gastric cancer, chondrosarcoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, esophageal cancer, gastroesophageal junction cancer, gastroesophageal adenocarcinoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, breast cancer. In certain embodiments, the cancer can be, for example, colorectal adenocarcinoma. In certain embodiments, the cancer can be, for example, small bowel adenocarcinoma. In certain embodiments, the cancer can be, for example, hepatocellular carcinoma. In certain embodiments, the cancer can be, for example, head and neck cancer. In certain embodiments, the cancer can be, for example, renal cell carcinoma. In certain embodiments, the cancer can be, for example, ovarian cancer. In certain embodiments, the cancer can be, for example, prostate cancer. In certain embodiments, the cancer can be, for example, lung cancer. In certain embodiments, the cancer can be, for example, uterine sarcoma. In certain embodiments, the cancer can be, for example, esophageal cancer. In certain embodiments, the cancer can be, for example, endometrial cancer. In certain embodiments, the cancer can be, for example, cholangiocarcinoma. In certain embodiments, each of the cancers can be, for example, unresectable, advanced, refractory, recurrent, or metastatic. A preferred cancer is a solid cancer, in particular hepatocellular carcinoma or colorectal cancer.

In **a second aspect,** the invention pertains an agonist of ferroptosis for use in the treatment of a proliferative disorder, in a subject, the treatment comprising exposing tissue or cells involved with the proliferative disorder in the subject to: (i) an immune checkpoint inhibitor, and (ii) the agonist of ferroptosis, and, optionally, (iii) a blocker of myeloid cell recruitment.

Preferably, the treatment of the second aspect further comprises exposing tissue or cells involved with the proliferative disorder in the subject to: (iii) an inhibitor of immune suppressor cells, such as preferably myeloid derived suppressor cells (MDSC). Such an inhibitor is preferably a blocker of MDSC.

Preferably, the treatment comprises that (i) and or (ii) are administered to the subject, preferably, wherein the MDSC inhibitor is also administered to the subject.

In one preferred embodiment, the inhibitor of immune suppressor cells is selected from an antagonist of one or more high-mobility group box 1 (HMGB-1), colony-stimulating factor 1 receptor (CSF-R), granulocyte colony-stimulating factor (G-CSF), chemokine (C-X-C motif) ligand 1 (CXCLi), C-C motif chemokine ligand 2 (CCL2), or C-X-C chemokine receptor type 2 (CXCR2) or CXCR4.

As used herein, the terms "CCL2 inhibitor" refers to compounds which inhibit signalling through CCL2, as well as compounds which inhibit the expression of the CCL2 gene. Compounds which inhibit signalling through CCL2 are called CCL2 antagonists. They include compounds which inhibit the activity of CCL2, by directly binding to CCL2, or by inhibiting CCL2 signalling by other mechanisms. CCL2 antagonists include, but are not limited to, small organic molecules, antibodies and aptamers. Typically, CCL2 antagonists are antibodies, monoclonal or polyclonal, and preferably a human antibodies, chimeric antibodies or humanized antibodies. As used herein, the term "inhibitor" refers to compounds which inhibit signalling through their respective target (such as HMGB-1, CSF-R, G-CSF, CXCL1, CCL2, CXCR2 or CXCR4, as well as compounds which inhibit the expression of the target gene. Compounds which inhibit signalling through CCR2 are called CCR2 antagonists. They include compounds which inhibit the activity of CCR2, for example by directly binding to CCR2 and by inhibiting the CCL2 signalling, or by inhibiting CCR2 signalling by other mechanisms. CCR2 antagonists include, but are not limited to, small organic molecules, antibodies and aptamers.

In **a third aspect,** the invention pertains to an agonist of ferroptosis for use in aiding a treatment with an immune checkpoint inhibitor in a subject, by enhancing a cell mediated immune response in cells or tissue involved with, or surrounding, a proliferative disorder. The use of the agonist of ferroptosis therefore sensitizes the subject to the treatment with the immune checkpoint inhibitor.

An immune checkpoint inhibitor is selected from an immunotherapy comprising (administration of) an antibody binding to an immune checkpoint molecule, a non-antibody peptide or a small molecule, targeting and inhibiting CTLA-4, PD-1 or PD-L1. The invention surprisingly discovered that inducing or enhancing ferroptosis sensitizes proliferative disorders to a treatment with an immune checkpoint inhibitor, in particular to inhibitors of the PD-1, PD-L1 axis.

It is in some embodiments preferred that the treatments of the invention further comprises the administration of an inhibitor of immune suppressor cells, such as preferably myeloid derived suppressor cells (MDSC).

A proliferative disorder is a tumour disease, preferably a tumour disease characterized by a reduced susceptibility to a therapy with an immune checkpoint inhibitor.

In context of the invention the subject treatable by the therapies of the invention is preferably distinguished as having a tumour that progressed, in particular relapsed, recurred or did not respond to, prior radiotherapy. For example the tumour disease is a reoccurring or relapsing tumour disease.

Any agonist or inhibitor of a therapeutic target defined in the disclosed invention, and preferably the agonist of ferroptosis, is a compound or composition selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero-bi-functional compound (such as a PROTAC or a HyT molecule); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

In **a fourth aspect,** the invention pertains to a method of treating a disease in a subject in need of the treatment, the method comprising the steps of administering to the subject a therapeutically effective amount of an agonist of ferroptosis, wherein the treatment involves agonising ferroptosis in the subject and sensitizing cells involved with the proliferative disorder to a cell mediated immune response.

In **a fifth aspect,** the invention pertains to a method of treating a proliferative disorder in a subject in need of the treatment, the method comprising the concomitant or sequential administration of (i) an agonist or inducer of ferroptosis, (ii) an immune checkpoint inhibitor, and, optionally (iii) a blocker of myeloid cell recruitment.

In a **sixth aspect,** the invention pertains to an immune checkpoint inhibitor for use in the treatment of a proliferative disorder in a subject, the treatment comprising the sequential or concomitant administration of the immune checkpoint inhibitor and an agonist of ferroptosis to the subject.

In a **seventh aspect,** the invention pertains to a combination or composition for use in the treatment of a proliferative disorder in a subject, the treatment comprising the sequential or concomitant administration of the combination or composition to the subject, wherein the immune checkpoint inhibitor and an agonist of ferroptosis to the subject.

To be used in therapy, the compounds and compositions of the invention may be formulated into a pharmaceutical composition appropriate to facilitate administration to animals or humans. The term "pharmaceutical composition" means a mixture of substances including a therapeutically active substance (such as an agonist or antagonist of the invention) for pharmaceutical use.

Accordingly, in an additional aspect, the invention relates to a pharmaceutical composition comprising one or more compounds of the invention, such as an agonist of ferroptosis and/or an immune checkpoint inhibitor, and a pharmaceutically acceptable carrier, stabiliser and/or excipient.

In a preferred embodiment, the pharmaceutical composition of the invention may comprise the combination therapeutics in accordance with the herein disclosed treatment regimens, or alternatively, the invention may comprise a combination of pharmaceutical compositions, wherein each pharmaceutical composition comprises one single component of the combinatorial therapeutics of the invention, such as preferably one composition comprising the agonist of ferroptosis and a and a pharmaceutically acceptable carrier, stabiliser and/or excipient; another pharmaceutical composition comprising an immune checkpoint inhibitor and a pharmaceutically acceptable carrier, stabiliser and/or excipient; and optionally another pharmaceutical composition comprising a blocker of myeloid suppressor cells and a pharmaceutically acceptable carrier, stabiliser and/or excipient. The compositions of the invention in this embodiment may be combined into one therapeutic kit.

By way of example, the pharmaceutical composition of the invention may comprise between 0.1% and 100% (w/w) active ingredient (for example, an agonist of ferroptosis), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor® EL (formerly Cremophor EL™; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminium monostearate and gelatin.

Cells, such as immune cells (eg CAR T cells) for use with the invention can be included in pharmaceutical formulations suitable for administration into the bloodstream or for administration directly into tissues or organs. A suitable format is determined by the skilled person (such as a medical practitioner) for each patient, tissue, and organ, according to standard procedures. Suitable pharmaceutically acceptable carriers and their formulation are known in the art (see, e.g. Remington's Pharmaceutical Sciences 16th edition, Osol, A. Ed., 1980). Such cells, when formed in a pharmaceutical composition, are preferably formulated in solution at a pH from about 6.5 to about 8.5. Excipients to bring the solution to isotonicity can also be added, for example, 4.5% mannitol or 0.9% sodium chloride, pH buffered with art-known buffer solutions, such as sodium phosphate. Other pharmaceutically acceptable agents can also be used to bring the solution to isotonicity, including, but not limited to, dextrose, boric acid, sodium tartrate, propylene glycol, polyols (such as mannitol and sorbitol) or other inorganic or organic solutes. In one embodiment, a media formulation is tailored to preserve the cells while maintaining cell health and identity. For example, a premixture including an aqueous solution of anticoagulant (ACD-A), an equal amount of dextrose (50%), and phosphate buffered saline (PBS), or the like is pre-mixed and aliquoted in a volume to typically match or approximate the cellular matrix or environment from which the cell was extracted from the tissue or organ.

In some embodiments, the pharmaceutical composition comprising an agonist of ferroptosis is in unit dose form of between 10 and 1000mg agonist of ferroptosis. In some embodiments, the pharmaceutical composition comprising an agonist of ferroptosis is in unit dose form of between 10 and 200mg agonist of ferroptosis. In some embodiments, the pharmaceutical composition comprising an ABP is in unit dose form of between 200 and 400mg agonist of ferroptosis. In some embodiments, the pharmaceutical composition comprising an agonist of ferroptosis is in unit dose form of between 400 and 600mg agonist of ferroptosis. In some embodiments, the pharmaceutical composition comprising an agonist of ferroptosis is in unit dose form of between 600 and 800mg agonist of ferroptosis. In some embodiments, the pharmaceutical composition comprising an agonist of ferroptosis is in unit dose form of between 800 and 100 mg agonist of ferroptosis. Similar doses are used for an immune checkpoint inhibitor in accordance with the invention.

Exemplary unit dosage forms for pharmaceutical compositions comprising agonist of ferroptosis are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

In accordance with all aspects and embodiments of the medical uses and methods of treatment provided herein, the effective amount administered at least once to a subject in need of treatment with a compound or composition of the invention is, typically, between about 0.01mg/kg and about 100mg/kg per administration, such as between about 1mg/kg and about 10mg/kg per administration. In some embodiments, the effective amount administered at least once to said subject of a compound or composition of the invention is between about 0.01mg/kg and about 0.1mg/kg per administration, between about 0.1mg/kg and about 1mg/kg per administration, between about 1mg/kg and about 5mg/kg per administration, between about 5mg/kg and about 10mg/kg per administration, between about 10mg/kg and about 50mg/kg per administration, or between about 50mg/kg and about 100mg/kg per administration.

For the prevention or treatment of disease, the appropriate dosage of a compound or composition of the invention (or a pharmaceutical composition comprised thereof) will depend on the type of disease to be treated, the severity and course of the disease, whether the compound or composition of the invention and/or pharmaceutical composition is administered for preventive or therapeutic purposes, previous therapy, the patient's clinical history, age, size/weight and response to the compound or composition of the invention and/or pharmaceutical composition, and the discretion of the attending physician. The compound or composition of the invention and/or pharmaceutical composition is suitably administered to the patient at one time or over a series of treatments. If such compound or composition of the invention and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

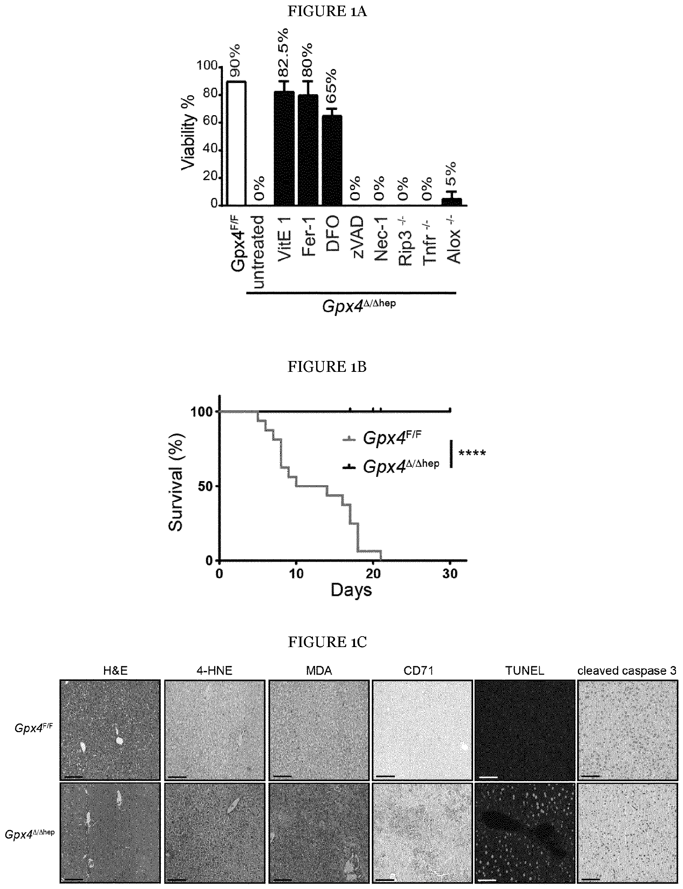

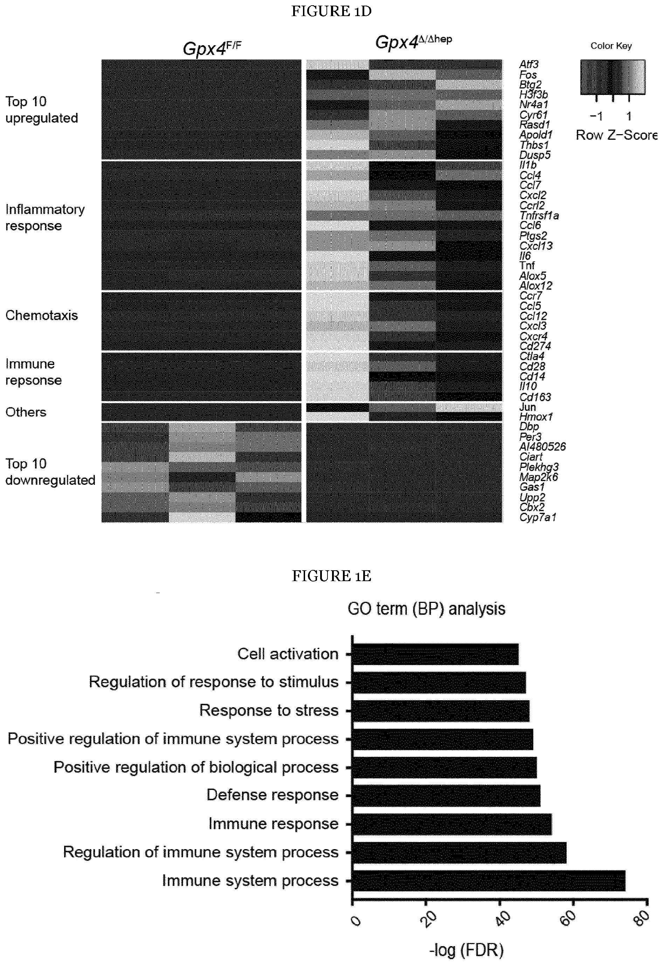

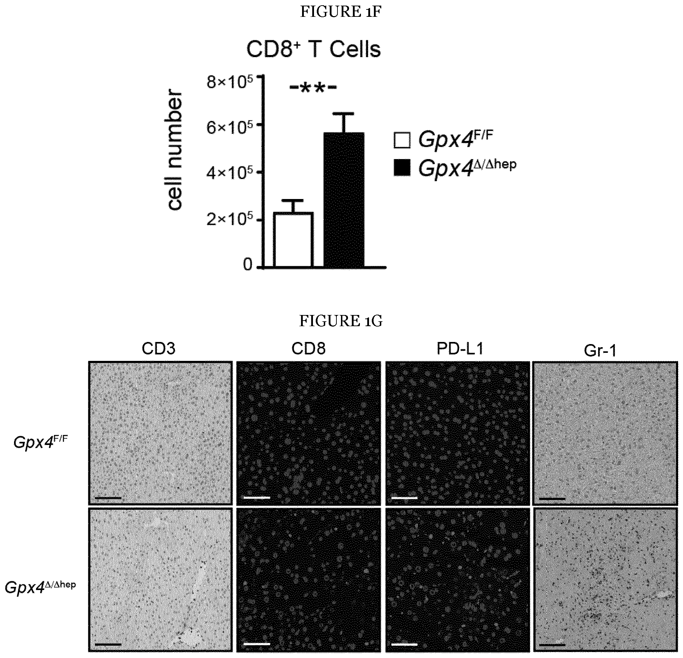

**Figure 1****:** shows **A,** Viability of primary hepatocytes from *Gpx4*^{F/F}, *Gpx4*^{Δ/Δ*hep*}, *Gpx4*^{Δ/Δhep}/*Rip3*^{-/-}, *Gpx4*^{Δ/Δhep}/*Tnfr1*^{-/-} and *Gpx4*^{Δ/Δhep}/*Alox*^{-/-} mice cultured for 24 hours *ex vivo* left either untreated or in the presence of vitamin E (VitE), ferrostatin-1 (Fer-1), deferoxamine (DFO), zVAD or necrostatin-1 (Nec-1). **B,** Survival of *Gpx4*^{F/F} and *Gpx4*^{Δ/ΔheP} mice placed on a vitamin E-depleted diet. N=9/genotype, ****p≤0,0001 by log-rank (Mantel-Cox) test. **C,** Representative H&E, 4-HNE, MDA, CD71, TUNEL and cleaved caspase 3 staining of livers from *Gpx4*^{F/F} and *Gpx4*^{Δ/Δhep} mice fed with vitamin E-depleted diet. **D,** Heatmap of the most differentially expressed genes in livers from *Gpx4*^{F/F} and *Gpx4*^{Δ/Δhep} mice fed with vitamin E-depleted diet. n=3/genotype. **E,** GOterm biological process analysis of *Gpx4*^{F/F} and *Gpx4*^{Δ/Δhep} mice fed with vitamin E-depleted diet. n=3/genotype. **F,** Flow cytometric analysis of CD8 T cells (Live⁺, CD45+, CD8+) in livers from *Gpx4*^{F/F} and *Gpx4*^{Δ/ΔheP} mice that were kept on vitamin E-depleted diet for two weeks. Data are mean ±SEM, n≥5, **p≤0,01 by Student's t test. **G,** Representative CD3, CD8, PD-Li and Gr-1 staining of livers from *Gpx4*^{F/F} and *Gpx4*^{Δ/ΔheP} mice fed with vitamin E-depleted diet.

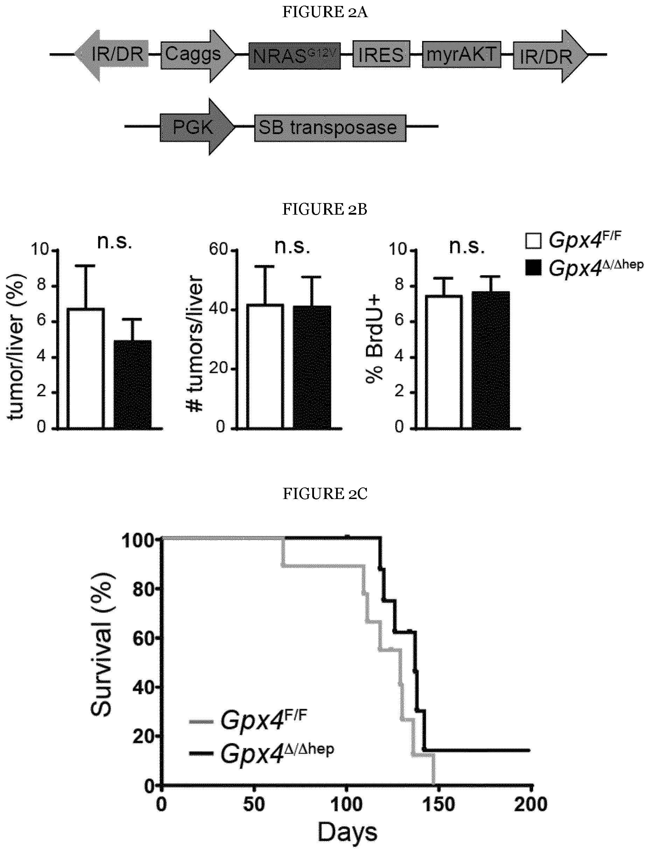

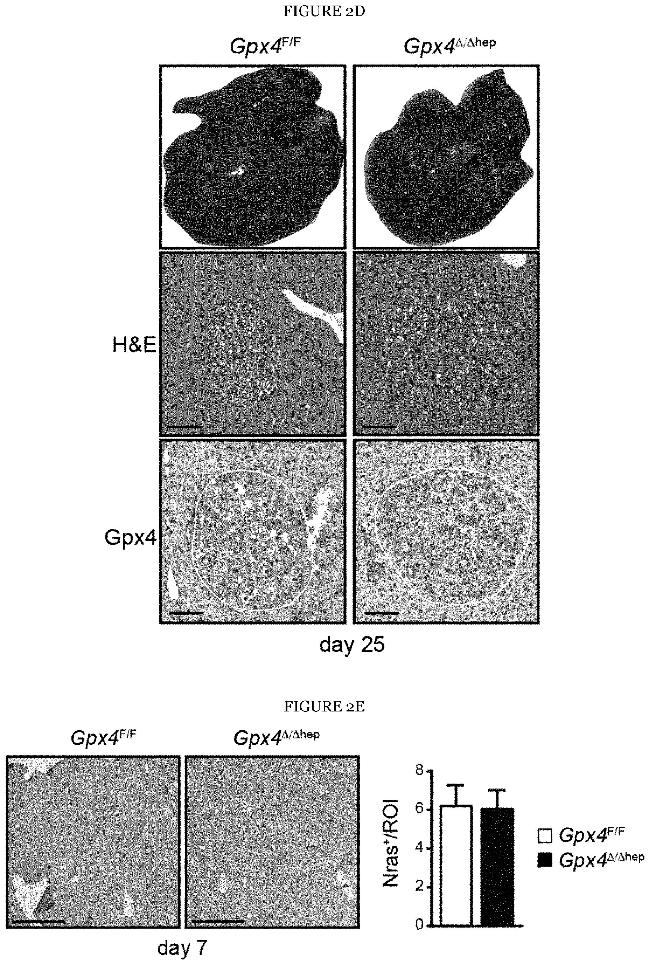

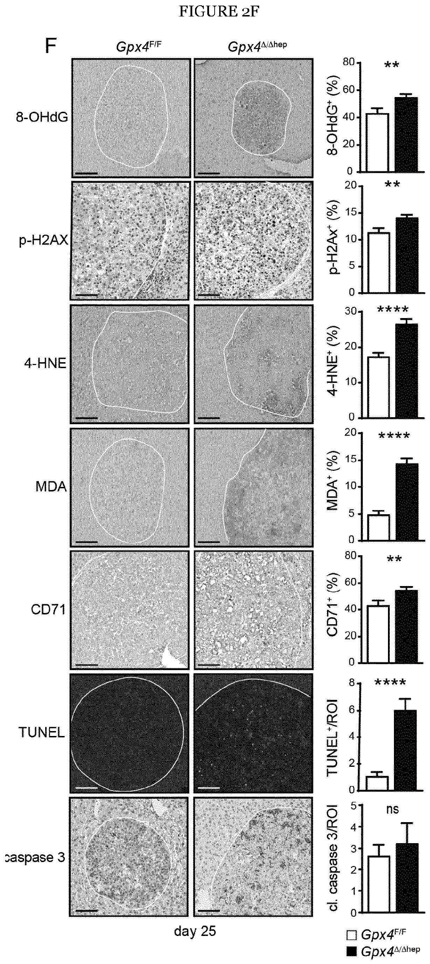

**Figure 2****:** shows **A,** Schematic representation of the two plasmids injected by hydrodynamic tailvein injection (HTVI) that contain transposable elements encoding oncogenic Nras^{G12V} and myristoylated AKT or sleeping beauty (SB) transposase. Caggs, CAGGS promoter; IR/DR, inverted repeats and direct repeats; IRES, internal ribosome entry site. **B,** Tumour incidence 25 days after HTVI quantified as the percentage of tumour area in the liver and the number of tumours per liver were analyzed on H&E stained serial sections. Tumour cell proliferation determined by immunohistochemical analysis of BrdU incorporation in livers. Data are mean ±SEM, n ≥ = 13, n.s.: not significant by Student's t test. **C,** Survival of *Gpx4*^{F/F} and *Gpx4*^{Δ/Δhep} mice with Nras/myrAKT induced liver tumours. N = 9/genotype, p= 0,176 by log-rank (Mantel-Cox) test. **D,** Representative macroscopic and microscopic appearance of liver tumours in *Gpx4*^{F/F} and *Gpx4*^{Δ/Δhep} mice 25 days after HTVI. Immunohistochemical staining of Gpx4 confirms absence of Gpx4 in *Gpx4*^{Δ/Δhep} hepatocytes and tumour cells. Scale bars = 100µm. **E,** Immunohistochemical analysis of Nras expression in livers of *Gpx4*^{F/F} and *Gpx4*^{Δ/Δhep} mice 7 days after HTVI. Data are mean ±SEM, n≥10. Scale bars = 100µm. **F,** Immunohistochemical analysis and quantification of 8-OHdG (n≥=73), p-H2AX (n≥82), 4-HNE (n≥99), MDA (n≥66), CD71 (n≥73) and cleaved-caspase 3 (n≥13) as well as TUNEL staining (n≥=35) in liver tumours of *Gpx4*^{F/F} and *Gpx4*^{Δ/Δhep} mice 25 days after HTVI. Data are mean ±SEM. *p≤0,05, **p≤0,01, ****p≤0,0001 by Student's t test. Scale bars = 100µm.

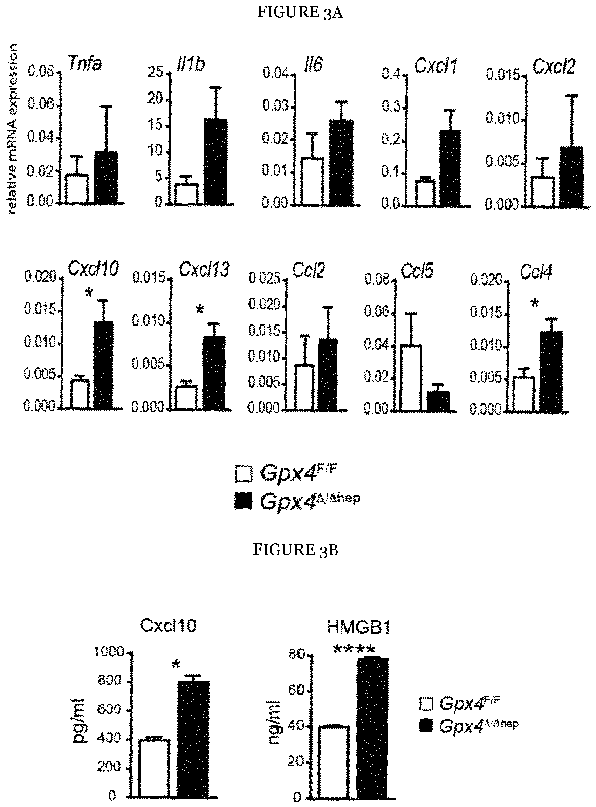

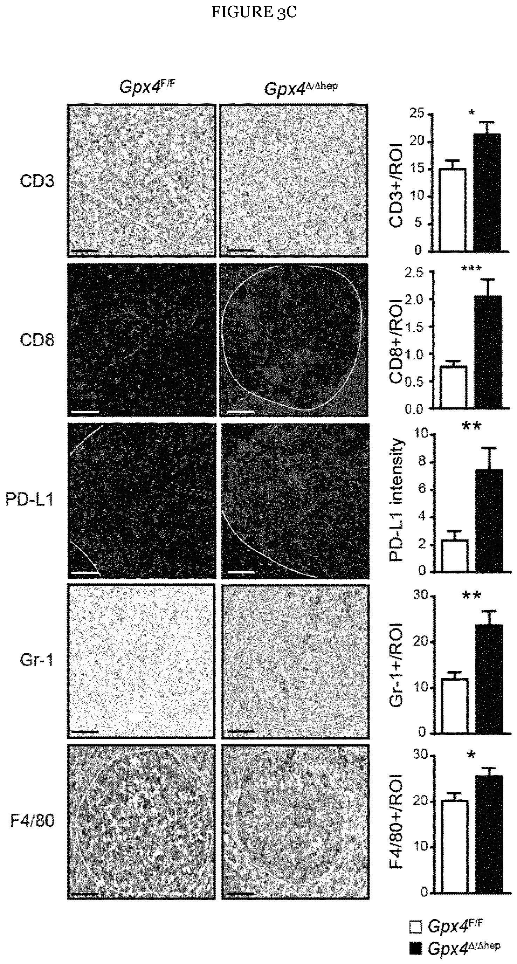

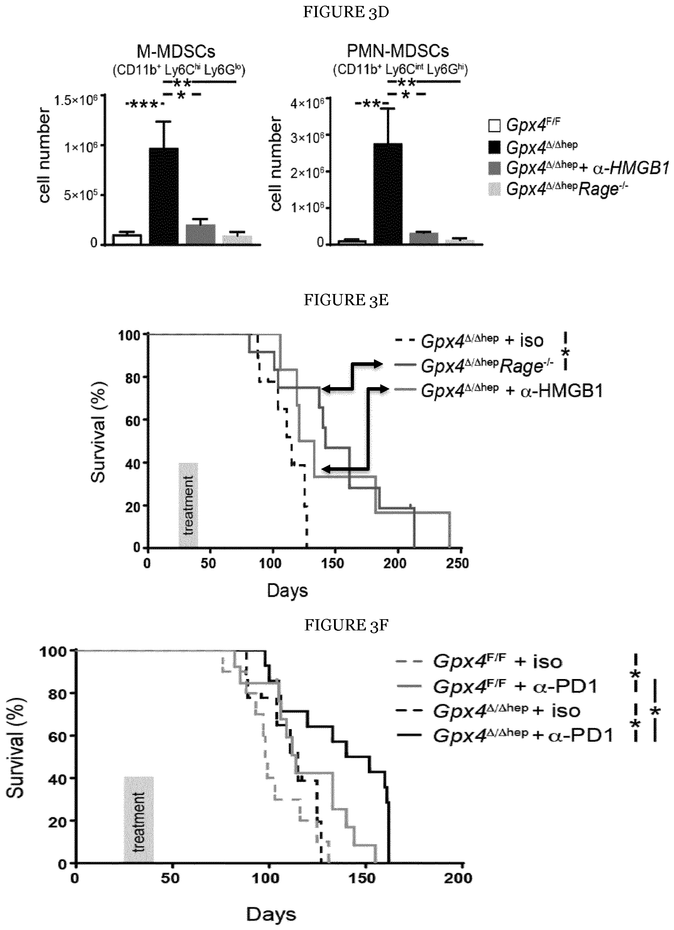

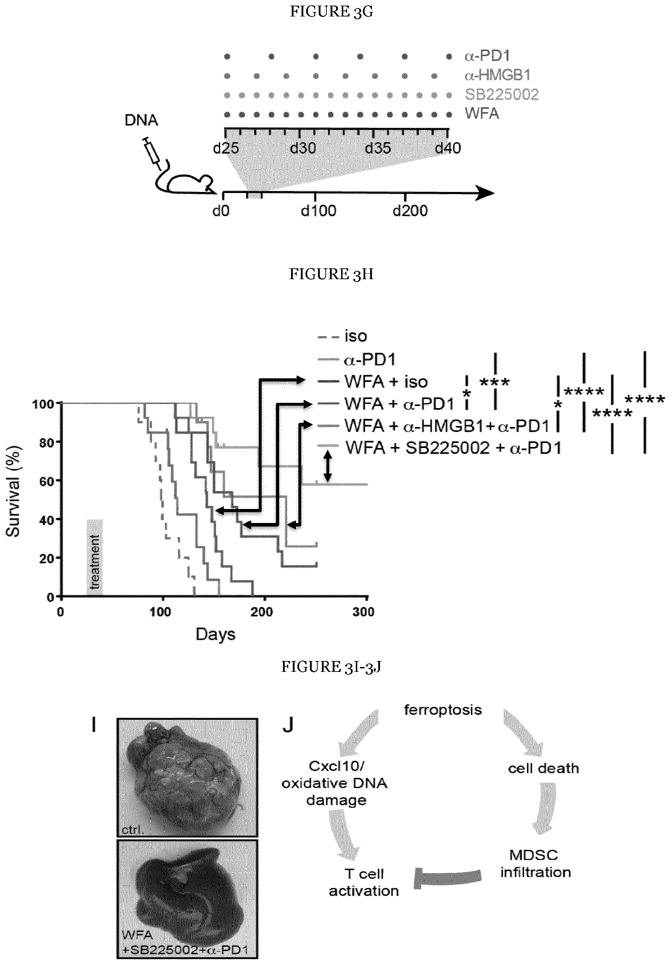

**Figure 3****:** shows **A,** Real-time qPCR analysis showing relative expression of the indicated genes in tumours from *Gpx4*^{F/F} and *Gpx4*^{Δ/ΔheP} mice 25 days after HTVI. Data are mean ±SEM, n=4/genotype, *p≤0,05 by Student's t test. **B,** CXCL10 and HMGB1 release by primary untransformed hepatocytes from *Gpx4*^{F/F} and *Gpx4*^{Δ/Δhep} mice cultured *ex vivo* for 4 hours. Data are mean ±SEM, n= 2/genotype. *p≤0,05 by Student's t test. **C,** Immunohistochemical analysis and quantification of CD3 (n=35), CD8 (n≥35), PD-Li (n=32), Gr-1 (n=15) and F4/80 (n=35) in liver tumours of *Gpx4*^{F/F} and *Gpx4*^{Δ/ΔheP} mice 25 days after HTVI. Data are mean ±SEM, number of tumours per genotype analyzed is indicated in brackets, *p≤0,05, **p≤0,01, ***p≤0,001 by Student's t test. Scale bars = 100µm. **D,** Flow cytometric analysis of Live⁺, CD45⁺, CD11b⁺, Ly6C^{hi}, Ly6G^{lo} (M-MDSC) and Live⁺, CD45⁺, CD11b⁺, Ly6C^{int}, Ly6G^{hi} (PMN-MDSC) in livers from mice that had been placed on a vitamin E-depleted diet for three weeks. *Gpx4*^{F/F} mice (n=7), *Gpx4*^{Δ/Δhep} mice (n=5) and *Gpx4*^{Δ/Δhep}/*Rage*^{-/-} (n=5) were left untreated or *Gpx4*^{Δ/Δhep} mice were injected daily with □-HMGB1(50µg/kg; n=2). Data are mean ±SEM. *p≤0,05, **p≤0,01, *** p≤0,001 by one-way ANOVA with Dunnett's multiple comparisons test with *Gpx4*^{Δ/Δhep} as reference. **E,** Survival of *Gpx4*^{Δ/Δhep} + isotype (Rat IgG2a,κ; n=9), *Gpx4*^{Δ/Δhep}*Rage^{-l-}* (n=12) and *Gpx4*^{Δ/Δhep} + α-HMGB1 (n=6) mice with Nras/myrAKT induced liver tumours. Treatment duration (day 25-40 after HTVI) is indicated by a grey area. *p≤0,05 by log-rank (Mantel-Cox) test. **F,** Survival of *Gpx4*^{*F*/*F*}+ isotype (Rat IgG2a,κ) (n=10), *Gpx4*^{F/F} + α-PD1 (n=13), *Gpx4*^{Δ/Δhep} + isotype (Rat IgG2a,K) (n=9) and *Gpx4*^{Δ/Δhep} +α-PD1 (n=14) mice with Nras/myrAKT induced liver tumours. Treatment duration (day 25-40 after HTVI) is indicated by a grey area. *p≤0,05 by log-rank (Mantel-Cox) test. **G,** Schematic overview of treatment schedule: between day 25 and day 40 after HTVI mice are treated i.p. with 250µg of α-PD1 or isotype (Rat IgG2a,κ) every 3 days, 50µg of α-HMGB1 every other day, 2,5mg/kg of Withaferin A (WFA) daily or 1mg/kg of SB225002 daily. Each dot represents one injection. **H,** Survival of WT mice with Nras/myrAKT induced liver tumours receiving the indicated single or combination therapies. Isotype (Rat IgG2a,κ) treatment, n=10; α-PD1 treatment, n=13; WFA + isotype (Rat IgG2a,κ) treatment, n=13; WFA + α-PD1 treatment, n=14; WFA + α-HMGB1 + α-PD1 treatment, n=12; WFA + SB225002 + α-PD1 treatment, n=14. *p≤0,05; *p≤0,01; ***p≤0,001 and ****p≤0,0001 by log-rank (Mantel-Cox) test. **I,** Macroscopic pictures of livers of untreated mice (upper picture) that have reached the endpoint or mice that have been treated with WFA+SB225002+α-PD1 >300 days after HTVI. **J,** Schematic summary: ferroptosis induces T cell recruitment to livers by Cxcl10 release and presumably oxidative DNA damage while it simultaneously triggers HMGBi-dependent MDSC infiltration, which suppresses T cell activation.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Gpx4 dependent ferroptosis induces immune gene expression in liver cells

Marked ferroptosis induction could be confirmed in primary *Gpx4*-deficient hepatocytes *ex vivo* (Fig. SiA), which can usually be compensated in *vivo* by vitamin E typically present in regular chow (*8*),(*9*). Indeed, mice with a hepatocyte-restricted Gpx4 deletion (*Gpx4*^{Δ/Δhep}) succumbed to liver failure within 21days when placed on a vitamin E-depleted diet, which was not observed when mice were kept on a regular chow (Fig. 1B). Gpx4-dependent liver damage was characterized by distinct zonal necrosis accompanied by sinusoidal congestion, hemorrhage and signs of immune cell infiltration (Fig. 1C). In line with ferroptosis induction in *vivo Gpx4*^{Δ/Δhep} livers were TUNEL positive and caspase3 negative and expressed CD71 (*10*) (Fig. 1C). Strikingly, RNAseq analysis and *GOterm* pathway analysis of livers from *Gpx4*^{F/F} and *Gpx4*^{Δ/Δhep} mice revealed a significant enrichment of genes connected to inflammatory and immune response pathways (Fig. 1D-E). Considering that many of these gene products are known to promote tumour development (*11*), it was reasoned that during tumourigenesis ferroptosis may counteract the previously suggested cell autonomous tumour suppression by creating a pro-inflammatory, pro-tumourigenic microenvironment.

### Example 2: Gpx4 loss during liver tumourigenesis

Thus, to examine the consequences of *Gpx4* loss during liver tumourigenesis, the inventors took advantage of a transposon-based mouse model of liver cancer (*9*),(*12*) that is achieved by hydrodynamic tail vein injection of a construct encoding for 2 oncogenes, Nras^{G12V} and myrAKT, together with a transposase encoding plasmid (Fig. 2A). Remarkably, tumour load, tumour cell proliferation and survival rates were comparable between *Gpx4*^{F/F} controls and *Gpx4*^{Δ/Δhep} mice (Fig. 2B-D). Accordingly, Nras immunostainings confirmed similar efficiency in transposon delivery after 7 days in both genotypes (Fig. 2E). However, histological analysis of early tumours revealed a significantly increased expression of the lipid peroxidation markers 4-HNE and MDA in *Gpx4* deficient tumour cells (Fig. 2F), which was associated with elevated levels of oxidative DNA damage (Fig. 2F). Moreover, liver tumours in *Gpx4*^{Δ/Δhep} mice were characterized by a striking increase in the number of TUNEL-positive cells while the number of apoptotic cells determined by cleaved caspase3 did not differ (Fig. 2F) supporting the notion that *Gpx4*-deficient tumour cells died from non-apoptotic, lipid peroxidation induced death, e.g. ferroptosis. In line with this notion, also the number of CD71⁺ cells was elevated (Fig. 2F). Thus, the increased hepatic tumour cell death did not lead to tumour suppression in *Gpx4*^{Δ/Δhep} mice.

### Example 3: Inhibition of immune checkpoints and suppressor cell recruitment in combination with ferroptosis induction

Yet, considering the inflammatory response associated with the pronounced ferroptosis in liver failure, the examined gene expression profiles on day 25 of the tumour model was analyzed and the inventors found a similar upregulation of several chemokines and cytokines including Cxcl10 (Fig. 3A) that is known to be a potent chemoattractant for various immune cells and particularly T cells. It was confirmed that ferroptotic hepatocytes secreted increased amounts of Cxcl10 and the inventors detected in addition a pronounced release of high-mobility group box 1 (HMGB-1) (Fig. 3B), which is involved in recruitment of myeloid derived suppressor cells (*13*). Immunofluorescence confirmed an increased number of CD3⁺ and CD8⁺ T cells in *Gpx4*^{Δ/Δhep} tumours (Fig. 2C), which, however, was accompanied by a marked upregulation of PD-Li in *Gpx4*-deficient tumour cells (Fig. 3C). Moreover, *Gpx4*^{Δ/Δhep} tumours were further characterized by an enhanced F4/80⁺ and Gr-1⁺ myeloid cell infiltration (Fig. 3C), the latter comprising potentially myeloid derived suppressor cells (MDSC). Remarkably, even in tumour-free *Gpx4*^{Δ/Δhep} mice hepatocyte ferroptosis led to CD8⁺ T cell infiltration and PD-Li upregulation (Fig. 1F, G) and a striking increase of M-MDSC and PMN-MDSCs (Fig. 3D). MDSC recruitment was indeed dependent on HMGB1 as deletion of *Rage* or treatment with an α-HMGB1 antibody (*14*) prevented their accumulation in *Gpx4*^{Δ/Δhep} mice with liver failure (Fig. 2D).

To confirm that HMGBi-dependent MDSC infiltration in *Gpx4*-deficient tumours also accounted for T cell suppression and consecutive tumour promotion, the inventors either induced HCC in *Gpx4*^{Δ/Δhep}/*Rage*^{-/-} compound mutants or treated tumour-bearing *Gpx4*^{Δ/Δhep} single mutant mice with HMGB1 neutralizing antibody for two weeks starting on day 25 when we detected immune cell infiltration (Fig. 3C). While α-HMGB1 antibody treatment shifted the median survival from 113 to 127 days (p = 0,0816), *Rage* deletion prolonged overall survival of *Gpx4*^{Δ/Δhep} mice significantly (Fig. 3E), yet in both cases some mice survived more than 200 days confirming the tumour promoting role of MDSC upon ferroptosis induction.

The pronounced T cell infiltration paralleled by PD-Li upregulation suggested that hepatocellular tumours in *Gpx4*^{Δ/Δhep} mice may be particularly amenable to checkpoint inhibition. Therefore, the inventors injected tumour carrying *Gpx4*^{F/F} controls and *Gpx4*^{Δ/Δhep} mice six times with an α-PD-1 antibody for a period of two weeks starting on day 25 of the model to adequately reflect patient treatment. Expectedly, blocking PD-1 prolonged survival significantly more in *Gpx4*^{Δ/Δhep} mice and shifted their median survival from 114 to 146 days (Fig. 3F) supporting our hypothesis that ferroptosis-induced T cell activation can be unleashed by targeting PD-1.

Considering the promising results when blocking PD-1 or MDSC recruitment to prolong survival of *Gpx4*^{Δ/Δhep} mice in the context of ferroptosis, the inventors wanted to examine whether pharmacological ferroptosis induction while targeting both immunosuppressive pathways, MDSCs and PD-1, could represent a therapeutic option for HCC treatment. To this end the inventors induced tumours in WT mice and treated with withaferin A, a natural anticancer agent recently shown to induce ferroptosis by lipid peroxidation in *vivo (15).* Additionally, mice were treated either with α-PD1, the α-HMGB1 neutralizing antibody or alternatively the CXCR2 inhibitor SB225002, a potent blocker of myeloid cell recruitment (*16*). Tumour bearing mice were treated for only two weeks starting on day 25 of the model (Fig. 3G). While single administration of PD-1 did not affect survival, withaferin A mono-treatment prolonged median survival by 44.5 days, yet the combination of these two was even more effective (Fig. 3H) further supporting the notion that ferroptosis sensitizes for checkpoint inhibition. However, when we in addition also blocked HMGB1 50% of mice survived more than 200 days (Fig. 3H). The best outcome was achieved when we combined withaferin A, α-PD-1 and SB225002, which led to survival of 60% of mice after 300 days. Indeed, at this late time point livers of these mice were completely tumour free (Fig. 3I). Collectively, these data emphasize the relevance of targeting MDSCs infiltration to yield the full potential of ferroptosis induction and α-PD-1 therapy. Importantly, the treatments, although given only over a period of two weeks, provided a long-lasting effect in this highly aggressive model.

Collectively, the invention demonstrates that hepatocyte ferroptosis triggers an immune cell-rich pro-inflammatory yet immunosuppressive tumour microenvironment. Therefore, the present invention challenges the concept of immunogenic cell death (*17*) in the context of ferroptosis. While ferroptotic hepatocytes trigger the enhanced recruitment of CD8⁺ T cells, PD-L1 upregulation in tumour cells possibly induced by the infiltrating CD8⁺ T cells that release IFNγ as well as HMGB-1 dependent myeloid cell recruitment both ultimately suppress T cell activation. Initial T cell recruitment is presumably dependent on Cxcl10 upregulation, which has been described in the context of cell death. However, unlike other modes of cell death, such as apoptosis or necroptosis, ferroptosis is associated with oxidative DNA damage, which may lead to formation of neo-antigens and may explain the good response to the combination of checkpoint inhibition and blockade of myeloid cell recruitment in ferroptotic livers. Thus, our study provides the basis for a new combinatorial immunotherapy approach for HCC treatment, and potentially other hard-to-treat cancers that are characterized by T cell exclusion and resistance to checkpoint inhibition.

### Materials and Methods

### Mice

Gpx4F/F (18), Tnfr1Δ (Jackson Laboratory, JAX:003242), Rip3Δ (19), Alox12/15A (20) mice and Rage-/- (21) mice were crossed to Alb-Cre mice (JAX:003574; Jackson Laboratory,) Mice were kept in a temperature-controlled room with 12 h light and 12 h dark diurnal cycle. They were housed in filter-topped cages and were standard laboratory chow and water ad libitum. All animal procedures were performed in accordance with institutional guidelines. No statistical method was used to predetermine sample size. Livers were photographed and sampled. Counting of tumours was done in a blinded fashion. Vitamin E-deficient diet was purchased from Ssniff (E15791-147).

### Isolation of primary murine hepatocytes

Mice were anaesthetized with ketamine/rompune (100 mg/200 mg per kg body weight). Afterwards a cannula was inserted in the vena cava inferior for perfusion of the liver. The isolation procedure was adapted from Godoy et al. (22). Briefly, perfusion was performed with 37°C warm HBSS without Ca2+ and Mg2+ (supplemented with 15 mM HEPES, 2.5 mM EDTA, 1 g/l glucose, 100 U/ml penicillin and 100 µg/ml streptomycin, 1% non-essential amino acids) using a roller pump (10 ml/min) for 10 min. Thereafter, the liver was perfused with HBSS with Ca2+ and Mg2+ (supplemented with 15 mM HEPES, 5 mM CaCl2, 0.13 mg/ml collagenase IV (Sigma-Aldrich, C5138, Darmstadt, Germany), 100 U/ml penicillin, and 100 µg/ml streptomycin] for additional 10 min. The liver was carefully removed from the abdominal cavity, placed in a Petri dish on ice in Williams E medium (supplemented with 10% FCS, 100 U/ml penicillin, and 100 µg/ml streptomycin) and opened with forceps. Liver cells were resuspended and put over a 100 µm cell strainer. After two rounds of centrifugation (5 min at 50 g and 4°C) and resuspension, cell viability was determined by trypan blue dye exclusion, and 200.000 cells were seeded per well (12-well plate) in Williams Medium E (supplemented with 10% FCS, 2 mM L-Alanyl-L-Glutamin (Biochrom), 100 U/ml penicillin, and 100 µg/ml streptomycin) on collagen coated plates (Roche collagen rat tail). Adherent hepatocytes were washed after 4 h with PBS and fresh Williams Medium E (supplemented with 10% FCS, 2 mM L-Alanyl-L-Glutamin (Biochrom), 100 U/ml penicillin, and 100 µg/ml streptomycin). For inhibitor treatment inhibitors were dissolved in medium and changed after 4 hours and every 24 hours.

### Inhibitor experiments

Vitamin E (T3251; Sigma), Necrostatin-1 (480065; Calbiochem), Ferrostatin-1(341494; Calbiochem), Deferroxamin (deferoxamine mesylate salt) (D9533; Sigma), Z-VAD (OMe)-FMK (Caspase Inhibitor-i) (627610; Calbiochem) were dissolved in DMSO, EtoH or PBS according to manufacturer suggestions and used in the described concentrations. For quantification of cell death, cells were trypsinized and stained with trypan blue followed by counting with a haemocytometer using standard protocol. Cells stained blue were considered as dead cells.

### Liver Tumour mouse model: Hydrodynamic tail vein injections

For transposon-mediated intrahepatic gene transfer mice received a 5:1 molar ratio of transposon- to transposase encoding vector (30 µg total DNA). DNA for hydrodynamic tail vein injection was prepared using Qiagen EndoFreeMaxi Kit (Qiagen, Hilden, Germany) and dissolved in 0.9% NaCl solution to a final volume of 10% of animal's body weight. Animals (5-7 weeks old) were injected within 10 s with 25 µg transposon plasmids and 5 µg transposase. Transposon-based vector pCAGGs-IRES was used.

### In vivo treatments

All treatments were administrated from day 25 until day 40 post hydrodynamic injection. 250µg of α-PD-1 (CD279) monoclonal antibody (clone RMP1-14, BioXCell) or IgG2a isotype control was administered i.p. every 3 days for six injections. 50µg of α-HMBG1 was injected i.p every other day for the tumour model and daily for the vitamin E depleted diet. Withaferin A (Sigma-Aldrich) and SB225002 (SelleckChem) were injected i.p. daily at 2,5mg/kg and 1mg/kg respectively. Some mice developed lesion on the ear, paws and mostly on the tails as described previously (9). Following our animal experimentation protocol, mice with lesion at eminent risk of rupture or bleeding were euthanized. Any mouse that was sacrificed due to those lesions or for any health reason unrelated to liver tumour was censured at the time of sacrifice.

### Histology and immunochemistry

For histological analysis tissue was fixed in 4% PFA and paraffin embedded. After deparaffinization and rehydration of the tissue haematoxylin and eosin (H&E) staining was done according to standard protocols. Tumour number, size and tumour to liver ratio quantification was performed manually on H&E stained liver sections scanned on a Aperio CS2 (Leica). To determine cell proliferation, mice were injected i.p. with 75 mg/kg BrdU (Sigma-Aldrich) 90 min before sacrifice. For MDA (6463; Abcam), 8-OHdG (10802; Abcam) and 4-HNE (46545; Abcam) staining, paraffin sections were incubated in proteinase K for up to 40 minutes at 37C° for antigen retrieval. Alkaline phosphatase reaction was used as a color reaction with nuclear fast red (Sigma) as a nuclear counter stain. For Nras staining (F-155; Santa Cruz) paraffin sections were incubated in proteinase K for up to 40 minutes at 37C° for antigen retrival and vector mouse on mouse Kit (MOM PK-2200; Vector) used for staining. For CD71 immunohistochemistry, slides were de-paraffized and subjected to heat-induced antigen retrieval in Tris-EDTA (pH:9.0) solution for 15 min. Slides were blocked and treated with transferrin receptor primary antibody (H68.4; Invitrogen) using MOM kit according to the manufacturer's instructions. Detection was performed with the DAB substrate kit (SK-4100; Vector). For α-F4/80 (6640; Abcam), GPx4 (125066; Abcam), α-BrdU (MCA2060; AbD Serotec), α-cleaved caspase 3 (9661S; Cell Signaling Technology), α-CD3 (IS503: DAKO), α-GR-1 (Ly6G) (11-5931-82; eBioscience), α-p-H2AX (9661; Cell Signaling Technologies) paraffin sections were stained using a Leica Bond Max following standards immunochemistry staining protocols. 4-HNE, MDA, p-H2AXa, CD71 and 8-OHdG stained sections were scanned using Aperio CS2 (Leica). Quantification of 4-HNE, MDA, and CD71 was done using a membrane algorithm whereas quantification of 8-OHdG and p-H2AX was done using a nuclear staining algorithm (Aperio eSlide manager software) on manually marked tumours. For CD3, Gr-1, F4-80 and cleaved caspase 3, non-overlapping images within the tumour area were acquired with 20x objective and positive cells were counted manually.

### Immunofluorescence

Terminal deoxynucleotidyl transferase deoxyuridine triphosphate nick-end labeling (TUNEL) assay was performed with the ApoAlert DNA Fragmentation Assay Kit (Clontech, 630107). Briefly, tissues were treated with 0,9% NaCl for 5 min, then with with 4% PFA for 5min followed by an incubation for 5min with proteinase K for 5min. After being blocked for 10-15 min, sections were incubated with terminal deoxynucleotidyl transferase enzyme mix for 1h and then mounted in medium containing 4,6 diamidino-2-phenylindole. For CD8 and PDL1 staining, Section were stained overnight with aPDLi (13684; Cell Signaling Technologies) or CD8 (361003; Synaptic Systems) after antigen retrieval with Citrate Buffer for 10min and followed by a AF594-coupled secondary antibody. Image acquisition was performed using a Zeiss Axio Imager M2 with a 20×/0.5 EC Plan Neofluar or a 40×/0.95 korr Apochromat objective. The images were captured on a Zeiss microscope and positive cells were quantified manually.

### RNA sequencing

RNA sequencing was done with the help of the "Transcriptome and Genome Analysis Laboratory (TAL), University of Göttingen, Germany. RNA was extracted via Trizol extraction protocol. Quality control was done by Agilent Bioanalyzer 2100. As starting material for the library preparation, 0.5µg of total RNA was used. The libraries were generated according to the TruSeq mRNA Sample Preparation Kits v2 Kit from Illumina (Cat. N°RS-122-2002). The fluorometric based QuantiFluor™ dsDNA System from Promega (Mannheim, Germany) was used for accurate quantitation of cDNA libraries. The size of final cDNA libraries was determined by using the Fragment Analyzer from Advanced Bioanalytical. cDNA libraries were amplified and sequenced by using the cBot and HiSeq4000 from Illumina (SR; 1×50bp; ca. 30 Mio reads per sample). Sequence images were transformed to bcl files using Illumina software BaseCaller, which were demultiplexed to fastq files with bcl2fastq and quality checks were done via fastqc. Sequences were aligned to the genome reference (mm9) using the STAR alignment software (version 2.3.0e) allowing for 2 mismatches within 50 bases. Subsequently, filtering of unique hits and counting were conducted with SAMtools (version 0.1.18) and HTSeq (version 0.6.1p1). Read counts were analyzed in the R/Bioconductor environment (version 3.2, www.bioconductor.org) using the DESeq2 package (version 1.8.1). Candidate genes were filtered to a minimum of 2-fold change and FDR-corrected p-value < 0.05. Gene ontology enrichment analysis: the gene ontology analysis was carried out by using DAVID/EASE 6.7 (Huang et al.).

### Quantitative PCR

RNA isolation was performed using RNeasy Mini Kit (Qiagen) according to the manufacturers' instructions. For the RNA isolation from tumours the tumour pieces were shock frozen after harvest. The concentration and purity of RNA was determined and 1 µg RNA was used for cDNA synthesis using Superscript II reverse transcriptase kit (Invitrogen). First, 1 µg RNA, 2.5 µM OligodT and 0.5mM dNTP-mix were incubated at 65°C for 5 minutes for denaturation. The mixture was cooled down on ice for 2 minutes and mixed with 1X reaction buffer, 5 mM DTT, 0.5 U RNaseOUT (Invitrogen) and 10 U Superscript II reverse transcriptase and incubated 60 minutes at 50°C. For quantitative PCR analysis a reaction was performed with SYBR-Green MasterMix (Roche) on a StepOnePlus Real Time PCR system (Applied Biosystems). Expression levels were normalized based on the levels of the housekeeping gene cyclophilin.

### Flow cytometry

For immune cell staining by flow cytometry, after PBS perfusion, livers were collected and digested twice with 1mg/ml of Collagenase IV (Sigma) for a total of 1h. The remaining organ pieces were mashed and cell suspensions were filtered through a 70µm cell strainer. Immune cells were enriched thanks to a 30%/40%/75% Percoll gradient with a 1700rpm 17min centrifugation. Immune cells were then washed and stained for 15 to 20 min on ice with prestained for 5 min with α-CD16/CD32 (14-0161-82; eBioscience) then with Fixable Viability Dye-eF780 (eBioscience), α-CD45-FITC (11-0451-85; eBioscience), α-CD11b-BV650 (56340; BD) α-CD11c-BV785 (563735; BD), α-Ly6G-BV605 (563005; BD), α-Ly6C-AF700 (128024; Biolegend), α-F4/80-PE (12-4801-82, eBioscience), or with Fixable Viability Dye-eF780 (ebioscience), α-CD45-BV786 (564225; BD), α-CD4-BuV496 (564667; BD), α-CD8-BV650 (563234; BD). Samples were then fixed with 1% PFA and acquired on a Fortessa and analyzed with FlowJo. M-MDSCs are define as Live+, CD45+, CD11b+, CD11c-, Ly6Chi, Ly6Glo and while PMN-MDSCs are define as Live+, CD45+, CD11b+, CD11c-, Ly6Cint, Ly6Ghi. CD8 T cells are gated as Live+, CD45+, CD8+, CD4-.

### ELISA

The HMGB1 (IBL International) and Cxcl10 (R&D) ELISAs were performed according to the manufacturers' instructions using supernatants of cultured primary hepatocytes for 4 hours. The color reactions were detected spectrophotometrically using a plate reader at 450 nm with a correction at 540nm.

### Statistical analysis

Statistical parameters including the exact value of n, dispersion and precision measures (mean ± SEM) and statistical significance and the tests used are reported in the figures and figure legends. GraphPad PRISM software was used for statistical analysis the data is entered as columns for single measurements and entered as grouped for multiple measurements such as tumour size per animal. Data is judged to be statistically significant based on unpaired, two-tailed t-test when comparing two samples, based on one-way ANOVA and Bonferroni's multiple comparison or Tukey's multiple comparison test when comparing multiple samples and based on Mann-Whitney test when comparing sets without a standard deviation. The p values were represented in the figures and indicated in the legends as * p < 0.05, ** p < 0.01, *** p < 0.001, **** p < 0.0001.

### REFERENCES

The references are:
1. F. Ursini, M. Maiorino, M. Valente, L. Ferri, C. Gregolin, Purification from pig liver of a protein which protects liposomes and biomembranes from peroxidative degradation and exhibits glutathione peroxidase activity on phosphatidylcholine hydroperoxides. Biochim Biophys Acta 710, 197-211 (1982).
2. S. J. Dixon et al., Ferroptosis: an iron-dependent form of nonapoptotic cell death. Cell 149, 1060-1072 (2012).
3. J. P. Friedmann Angeli et al., Inactivation of the ferroptosis regulator Gpx4 triggers acute renal failure in mice. Nat Cell Biol 16, 1180-1191 (2014).
4. I. Ingold et al., Selenium Utilization by GPX4 Is Required to Prevent Hydroperoxide-Induced Ferroptosis. Cell 172, 409-422.e421 (2018).
5. J. P. Friedmann Angeli, D. V. Krysko, M. Conrad, Ferroptosis at the crossroads of cancer-acquired drug resistance and immune evasion. Nat Rev Cancer 19, 405-414 (2019).
6. L. Jiang et al., Ferroptosis as a p53-mediated activity during tumour suppression. Nature 520, 57-62 (2015).
7. W. Wang et al., CD8+ T cells regulate tumour ferroptosis during cancer immunotherapy. Nature 569, 270-274 (2019).
8. Y. Zhang et al., BAP1 links metabolic regulation of ferroptosis to tumour suppression. Nat Cell Biol 20, 1181-1192 (2018).
9. C. M. Carlson, J. L. Frandsen, N. Kirchhof, R. S. McIvor, D. A. Largaespada, Somatic integration of an oncogene-harboring Sleeping Beauty transposon models liver tumour development in the mouse. Proc NatlAcad Sci USA 102, 17059-17064 (2005).
10. H. Feng et al., Transferrin Receptor Is a Specific Ferroptosis Marker. Cell Rep 30, 3411-3423.e3417 (2020).
11. F. R. Greten, S. I. Grivennikov, Inflammation and Cancer: Triggers, Mechanisms, and Consequences. Immunity 51, 27-41 (2019).
12. R. Rudalska et al., In vivo RNAi screening identifies a mechanism of sorafenib resistance in liver cancer. Nat Med 20, 1138-1146 (2014).
13. J. Li et al., HMGB1 promotes myeloid-derived suppressor cells and renal cell carcinoma immune escape. Oncotarget 8, 63290-63298 (2017).
14. S. Qin et al., Role of HMGB1 in apoptosis-mediated sepsis lethality. J Exp Med 203, 1637-1642 (2006).
15. B. Hassannia et al., Nano-targeted induction of dual ferroptotic mechanisms eradicates high-risk neuroblastoma. J Clin Invest 128, 3341-3355 (2018).
16. S. L. Highfill et al., Disruption of CXCR2-mediated MDSC tumour trafficking enhances anti-PDi efficacy. Sci Transl Med 6, 237ra267 (2014).
17. L. Galluzzi, A. Buque, O. Kepp, L. Zitvogel, G. Kroemer, Immunogenic cell death in cancer and infectious disease. Nat Rev Immunol 17, 97-111 (2017).
18. A. Seiler et al., Glutathione peroxidase 4 senses and translates oxidative stress into 12/15-lipoxygenase dependent- and AIF-mediated cell death. Cell Metab 8, 237-248 (2008).
19. K. Newton, X. Sun, V. M. Dixit, Kinase RIP3 is dispensable for normal NF-kappa Bs, signaling by the B-cell and T-cell receptors, tumour necrosis factor receptor 1, and Toll-like receptors 2 and 4. Mol Cell Biol 24, 1464-1469 (2004).
20. D. Sun, C. D. Funk, Disruption of 12/15-lipoxygenase expression in peritoneal macrophages. Enhanced utilization of the 5-lipoxygenase pathway and diminished oxidation of low density lipoprotein. J Biol Chem 271, 24055-24062 (1996).
21. B. Liliensiek et al., Receptor for advanced glycation end products (RAGE) regulates sepsis but not the adaptive immune response. J Clin Invest 113,1641-1650 (2004).
22. P. Godoy et al., Recent advances in 2D and 3D in vitro systems using primary hepatocytes, alternative hepatocyte sources and non-parenchymal liver cells and their use in investigating mechanisms of hepatotoxicity, cell signaling and ADME. Arch Toxicol 87, 1315-1530 (2013).

## Claims

1. **An agonist of ferroptosis for use in the treatment of a proliferative disorder** in a subject, wherein the treatment involves (i) agonising ferroptosis by administrating to the subject the agonist of ferroptosis, and (ii) sensitizing cells involved with the proliferative disorder to a cell-mediated immune response.

2. The agonist of ferroptosis for use of claim 1, wherein the agonist of ferroptosis is a compound selected from the group consisting of (i) RSL3 and/or other GPX4 inhibitor or antagonist (such as withaferin A(WA)), (ii) erastin (e.g., and/or another compound which inhibits amino acid transporters system xc-), and (iii) buthionine sulfoximine (BSO) which inhibits gamma-glutamylcysteine synthetase and production of glutathione, or (iv) activators of the nuclear factor-like 2 pathway, for example through targeting of Kelch-like ECH-associated protein 1.

3. The agonist of ferroptosis for use of claim 1 or 2, wherein (ii) sensitizing cells involved with the proliferative disorder to a cell-mediated immune response involves the administration of an immune checkpoint inhibitor to the subject.

4. The agonist of ferroptosis for use of claim 3, wherein the immune checkpoint inhibitor is selected from an immunotherapy comprising (administration of) an antibody binding to an immune checkpoint molecule, a non-antibody peptide or a small molecule, targeting and inhibiting CTLA-4, PD-1 or PD-L1.

5. The agonist of ferroptosis for use of any one of claims 1 to 4, wherein the treatment further comprises (iii) supressing the recruitment of immune suppressing cells, such as by administering to the subject a blocker of myeloid cell recruitment.

6. The agonist of ferroptosis for use of claim 5, wherein the myeloid cell is a myeloid derived suppressor cell (MDSC).

7. The agonist of ferroptosis for use of claim 5 or 6, wherein supressing the recruitment of immune suppressing cells constitutes the administration to the subject of a compound selected from an antagonist of one or more high-mobility group box 1 (HMGB-1), colony-stimulating factor 1 receptor (CSF-R), granulocyte colony-stimulating factor (G-CSF), chemokine (C-X-C motif) ligand 1 (CXCLi), C-C motif chemokine ligand 2 (CCL2), or C-X-C chemokine receptor type 2 (CXCR2) or CXCR4.

8. **An agonist of ferroptosis for use in aiding a treatment with an immune checkpoint inhibitor** in a subject, by enhancing susceptibility of cells or tissue involved with a proliferative disorder to the treatment with the immune checkpoint inhibitor.

9. The agonist of ferroptosis for use of claim 8, wherein the immune checkpoint inhibitor is selected from an immunotherapy comprising (administration of) an antibody binding to an immune checkpoint molecule, a non-antibody peptide or a small molecule, targeting and inhibiting CTLA-4, PD-1 or PD-L1.

10. The agonist of ferroptosis for use of claim 13 or 14, wherein the treatment further comprises the administration of an inhibitor of immune suppressor cells, such as preferably myeloid derived suppressor cells (MDSC).

11. The agonist of ferroptosis for use of any one of the preceding claims, wherein the proliferative disorder is a tumour disease, a solid tumour disease **characterized by** a reduced susceptibility to a therapy with an immune checkpoint inhibitor.

12. The agonist of ferroptosis for use of any one of the preceding claims, wherein the proliferative disorder is a solid tumour, preferably hepatocellular carcinoma (HCC).

13. **An immune checkpoint inhibitor for use in the treatment of a proliferative disorder** in a subject, wherein the treatment comprises the concomitant or sequential induction of ferroptosis in the subject, preferably by administering to the subject an agonist of ferroptosis.

14. The immune checkpoint inhibitor for use of claim 13, which is selected from an inhibitor of PD-1 or PD-L1, such as an anti-PD-Li antibody.

15. The immune checkpoint inhibitor for use of claim 13 or 14, wherein the treatment further comprises the administration of an inhibitor of immune suppressor cells, such as preferably myeloid derived suppressor cells (MDSC).
